# EUROPÄISCHE PATENTANMELDUNG

(11) **EP 1 621 164 A1**
(43) Veröffentlichungstag der Anmeldung: **01.02.2006**
(21) Anmeldenummer: 05009267.5
(22) Anmeldetag: 28.04.2005
(51) Int. Cl.: A61F 13/08, D04B 1/00

(54) **Strickware zum Stützen und/oder Komprimieren und/oder Kompressionsbehandlung von Körperteilen**

(30) Priorität: 27.07.2004 DE 102004036344
(71) Anmelder: BSN -Jobst GmbH, 46446 Emmerich (DE)
(72) Erfinder: Gebel, Etienne, 46399 Bocholt (DE); Greve, Jürgen, 46446 Emmerich (DE); Krimmel, Gerhard, 72766 Reutlingen (DE)
(74) Vertreter: Kohler Schmid Möbus

(57) **Zusammenfassung**

Eine Strickware (10) zum Stützen und/oder Komprimieren von Körperteilen und/oder zur Kompressionsbehandlung sowie ein Verfahren zur Herstellung einer solchen Strickware, wobei die Strickware (10) mindestens bereichsweise aus mindestens einem elastischen Strickfaden und mindestens einem elastischen Schussfaden auf einer Flachstrickmaschine als Schlauch (11, 12, 13, 14, 15, 16) gestrickt wird.

## Beschreibung

Insbesondere zur Vermeidung oder Behandlung von Ödemen, Varicosen, venöser Insuffizienz sowie nach venenchirurgischen Eingriffen werden Kompressionsartikel wie Kompressionsstrümpfe, Kompressionshandschuhe, Fußkappen und dergleichen eingesetzt. Weitere elastische Artikel sind Gelenkbandagen oder auch Verbrennungsbandagen. Im Sport werden stützende Bandagen zum Schutz vor Verletzungen und teilweise auch zur Leistungserhöhung eingesetzt. Diese Artikel werden wegen der guten elastischen Eigenschaften vorwiegend als Gestricke hergestellt. Eine Möglichkeit besteht dabei darin, die Artikel auf einer Rundstrickmaschine zu fertigen. Auf Rundstrickmaschinen können Röhren konstanten oder begrenzt variablen Durchmessers gefertigt werden. Der Vorteil dieser Röhren besteht darin, dass diese keine, gegebenenfalls Druckstellen erzeugende Nähte aufweisen. Andererseits können die rund gestrickten elastischen Artikel aber nicht immer optimal den anatomischen Gegebenheiten des zu stützenden oder komprimierenden Körperteils angepasst werden. Sowohl die Passform als auch die gewünschte Wirkung rund gestrickter Artikel sind daher insbesondere bei extremen Anatomien nicht immer optimal.

Bei einer alternativen Fertigungsmöglichkeit werden die Artikel aus einem oder mehreren, auf einer Flachstrickmaschine gefertigten Teilen hergestellt. Auf einer Flachstrickmaschine ist es durch nadelgenaues Mindern und Zunehmen sowie durch Anwendung von Spickeltechniken relativ einfach möglich, auch sehr kompliziert geformte Konturen herzustellen. So lässt sich beispielsweise ein Handschuh recht gut als flächiges Gebilde der tatsächlichen Handform anpassen. Allerdings müssen die auf einer Flachstrickmaschine hergestellten Einzelteile später zusammengenäht werden. Dies verteuert die Fertigung der Artikel und führt außerdem zu Nähten, die den Tragekomfort durch Ausbildung von Druckstellen beeinträchtigen können. Nähte sind außerdem häufig diejenigen Stellen an einem Gestrick, die bei Belastungen des Gestricks während des Tragens sich teilweise wieder auftrennen können. Sie können somit die Lebensdauer und Optik des Gesamtgestricks beeinträchtigen.

Der vorliegenden Erfindung liegt die Aufgabe zugrunde, eine Strickware zum Stützen und/oder Komprimieren von Körperteilen und/oder zur Kompressionsbehandlung zu schaffen, die einen hohen Tragekomfort und eine hohe Lebensdauer aufweist und die kostengünstig zu fertigen ist.

Die Erfindung wird durch eine Strickware zum Stützen und/oder Komprimieren von Körperteilen und/oder zur Kompressionsbehandlung gelöst, die dadurch gekennzeichnet ist, dass sie mindestens bereichsweise aus mindestens einem elastischen Strickfaden und mindestens einem elastischen Schussfaden auf einer Flachstrickmaschine als Schlauchgestrick hergestellt ist Außerdem wird die Aufgabe durch ein Verfahren zur Herstellung einer solchen Strickware gelöst.

Die Herstellung von Schlauchgestricken auf Flachstrickmaschinen ist prinzipiell von der Fertigung von Bekleidungsstücken wie Pullovern und dergleichen bekannt. Es müssen dort jedoch keine elastischen Strickfäden und insbesondere keine elastischen Schussfäden verarbeitet werden. Die Verarbeitung elastischer Strickfäden und elastischer Schussfäden zur Herstellung von Schlauchgestricken auf einer Flachstrickmaschine ist zur Herstellung der erfindungsgemäßen Strickware erstmals unter Überwindung von Vorurteilen der Fachwelt gelungen. Die erfindungsgemäßen Strickwaren zeichnen sich dadurch aus, dass sie vollständig nahtlos und optimal an die Anatomie des betreffenden Körperteils angepasst sind. Durch den Schussfadeneintrag können die Elastizitätseigenschaften der Strickware exakt gesteuert werden. Dadurch ist es auch möglich, bestimmte Bereiche der Strickware mit anderen Elastizitätseigenschaften auszustatten als andere Bereiche. Durch die Möglichkeit, die Strickware als ein-, zwei- oder dreidimensionales Gebilde zu fertigen, das der Form des zu stützenden und/oder komprimierenden Körperteils anatomisch angepasst ist, entstehen somit Strickwaren mit einem bisher nicht erreichten Tragekomfort, einer verbesserten Passform und einer bisher nicht erreichten Präzision in der gewünschten Kompressions- und/oder Stützfunktion.

Die Strickware kann mindestens bereichsweise als Schlauch-Rund-Gestrick hergestellt sein, wobei sich dieses Gestrick dadurch auszeichnet, dass der Strickfaden und der Schussfaden spiralförmig Maschenreihe für Maschenreihe bilden, sodass auf allen Seiten des Schlauchs identische und ununterbrochene Elastizitäts- und Druckverhältnisse entstehen.

Es besteht jedoch auch die Möglichkeit, die Strickware mindestens bereichsweise aus zwei an den Rändern miteinander auf der Flachstrickmaschine verbundenen Strickteilen herzustellen. Hierdurch entstehen optisch sichtbare seitliche Verbindungslinien, die jedoch nicht auftragen und die insbesondere nicht wie Nähte gesonderte Arbeitsgänge zu ihrer Herstellung erfordern. Aus optischen oder auch funktionalen Gründen kann die Ausbildung solcher Pseudonähte bei der Strickware durchaus erwünscht sein.

Zur Herstellung der Strickwaren können verschiedene Bindungstechniken eingesetzt werden. Bevorzugt kann eine 1:1-Bindung verwendet werden.

Die Strickware kann mittels Strick- und Schussfäden mit einer Elastizität zwischen 1 % und 400 % und einer Feinheit von vorzugsweise 44 bis 3.000 Dtex hergestellt werden. Dabei können die Elastizitäten und Feinheiten der Strickfäden und der Schussfäden unterschiedlich sein.

Zur optimalen Anpassung der Strickware an die anatomischen Gegebenheiten der Körperteile, bei denen die Strickware Anwendung finden soll, können beispielsweise Spickel- und Umhängetechniken oder aber auch eine Variation der Maschengröße eingesetzt werden. Auch durch eine Variation der Fadenspannung lassen sich Unterschiede im Durchmesser der Schlauchgestricksbereiche und natürlich auch Variationen in den Elastizitäts- und Kompressionseigenschaften herstellen.

Eine weitere Erhöhung des Nutzens der Strickware kann dadurch erzielt werden, dass sie mit angestrickten Applikationen wie Taschen, Ösen, Öffnungen und dergleichen versehen ist. Dadurch erhält die Strickware einen zusätzlichen Nutzen ohne einen zusätzlichen Konfektionsaufwand.

Weitere Vorteile lassen sich erzielen, wenn die Strickware Bereiche mit unterschiedlichen Elastizitäts- oder Kompressionseigenschaften und/oder anderen Festigkeiten und/oder anderen Materialdicken aufweist. Gerade bei der Herstellung von Handschuhen ist dies von besonderer Bedeutung. So kann beispielsweise die Handinnenfläche zur Erhöhung der Scheuerfestigkeit aus anderen Materialien oder auch in einer anderen Materialstärke als die Handschuhrückhand hergestellt werden. Insbesondere lassen sich auch die Gestrickabschlüsse mit anderen Elastizitäts- oder Kompressionseigenschaften ausstatten als die übrigen Bereiche. Somit können Einschnürungen im Bereich der Gestrickabschlüsse vermieden werden. Die Gestrickabschlüsse können auch in einer Rippstruktur gefertigt werden, wodurch eine Rollneigung der Abschlüsse vermieden wird. Auch eine Ketteltechnik kann bei der Herstellung der Gestrickabschlüsse zur Anwendung kommen. Insbesondere bei Bein- oder Armstrümpfen ist es außerdem von Vorteil, die oberen Gestrickabschlüsse auf der Innenseite mit eingestrickten Noppen aus Elasthan- bzw. Elastodienfäden zu versehen, um ein Rutschen des Strumpfes zu verhindern.

Die erfindungsgemäße Strickware kann alle erdenklichen Formen aufweisen und für praktisch alle Körperteile eingesetzt werden. Bevorzugte Ausgestaltungen der Strickware sind Kompressionsarm- oder -beinstrümpfe, Kompressionshandschuhe, Kompressionsfusskappen oder Kompressionsstrumpfhosen. Die Strickware kann aber auch eine Verbrennungsbandage oder eine Sportbandage sein. Außerdem ist auch die Herstellung kompletter Anzüge, beispielsweise für Astronauten oder für Sportler zur Leistungsförderung, möglich.

Zur optimalen Anpassung der Strickware an den Körper des Trägers der Strickware kann die Form der Körperteile mittels eines Body-Scanning-Verfahrens erfasst und die Flachstrickmaschine mit den Daten über die Körperteilformen angesteuert werden. Dadurch lassen sich optimal auf die betreffende Person passende Artikel herstellen.

Für die Einbindung des die Elastizitäts- und Kompressionseigenschaften maßgeblich mit beeinflussenden elastischen Schussfadens gibt es verschiedene Möglichkeiten. Bei einem bevorzugten Herstellungsverfahren für die Strickware kann der mindestens eine elastische Schussfaden durch Fang in die Strickware eingebunden werden.

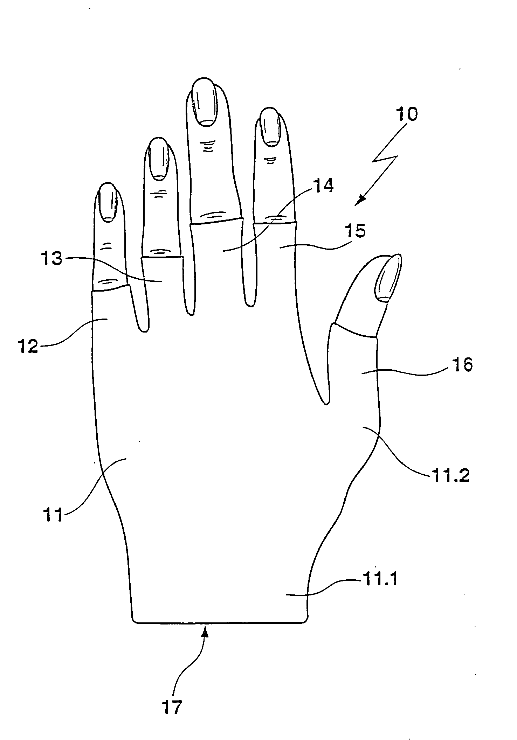

Nachfolgend wird anhand der Zeichnung eine mögliche Ausgestaltung einer erfindungsgemäßen Strickware näher beschrieben.

Die einzige Figur zeigt einen Kompressionshandschuh 10, der auf einer Flachstrickmaschine aus insgesamt sechs Schlauchgestricken 11 bis 16 gefertigt ist. Der Schlauch 11 großen Durchmessers umhüllt die eigentliche Hand, während die Schläuche 12 bis 16 die fünf Finger der Hand umschließen. Sämtliche Schläuche sind in sich nahtlos und nahtlos untereinander verbunden. Dies bedeutet, dass nirgends Nähte vorhanden sind, die den Tragekomfort einschränken könnten und die die Lebensdauer des Gesamthandschuhs 10 wegen einer erhöhten Verschleißanfälligkeit beeinträchtigen würden. Weiterhin zeigt die Figur mit dem Kompressionshandschuh 10, dass insbesondere der Schlauch 11 exakt der Anatomie der Hand angepasst ist. Dies bedeutet, dass im Bereich des Bündchens 11.1 der Schlauch 11 einen geringeren Durchmesser aufweist als im Bereich 11.2 des Daumengelenks. Dies wird dadurch erreicht, dass die Anzahl der Maschen im Bereich 11.2 gegenüber dem Bereich 11.1 erhöht wird. Dazu können Spickel- und/oder Umhängetechniken eingesetzt werden. Eine weitere Möglichkeit, eine Anpassung des Handschuhs 10 an die Anatomie der Hand zu erreichen, besteht darin, die Maschengrößen oder auch die Fadenspannung zu variieren. Durch diese Maßnahmen lassen sich außerdem die Elastizitätseigenschaften des Handschuhs 10 in einzelnen Bereichen variieren. Selbstverständlich kann auch die Strickbindung, mit der der Handschuh 10 hergestellt wird, in einzelnen Bereichen unterschiedlich gewählt werden. So können beispielsweise im Bereich des Handschuhbündchens 17 Rippen gestrickt werden, wodurch eine besonders hohe Elastizität erreicht und außerdem ein Aufrollen des Bündchens 17 vermieden wird. Wird der Handschuh 10 außerdem bei der Durchführung von Arbeiten mit der Hand eingesetzt, so kann die Handinnenseite mit einer erhöhten Scheuerfestigkeit ausgestattet werden. Dies kann durch eine geeignete Materialwahl der Strickfäden, eine Variation der Materialdicke oder eine entsprechende Wahl der Strickbindung erfolgen.

## Patentansprüche

1. Strickware (10) zum Stützen und/oder Komprimieren von Körperteilen und/oder zur Kompressionsbehandlung, **dadurch gekennzeichnet, dass** sie mindestens bereichsweise aus einem elastischen Strickfaden und mindestens einem elastischen Schussfaden auf einer Flachstrickmaschine als Schlauchgestrick (11, 12, 13, 14, 15, 16) hergestellt ist.

2. Strickware (10) nach Anspruch 1, **dadurch gekennzeichnet, dass** sie ein ein-, zwei- oder dreidimensionales Gebilde ist, das der Form des zu stützenden und/oder komprimierenden Körperteils anatomisch angepasst ist.

3. Strickware (10) nach Anspruch 1 oder 2, **dadurch gekennzeichnet, dass** sie mindestens bereichsweise als Schlauch-Rund-Gestrick (11, 12, 13, 14, 15, 16) hergestellt ist.

4. Strickware (10) nach Anspruch 1 oder 2, **dadurch gekennzeichnet, dass** sie mindestens bereichsweise aus zwei an den Rändern miteinander auf der Flachstrickmaschine verbundenen Strickteilen hergestellt ist.

5. Strickware (10) nach einem der Ansprüche 1 bis 4, **dadurch gekennzeichnet, dass** sie in einer 1:1-Bindung hergestellt ist.

6. Strickware (10) nach einem der Ansprüche 1 bis 5, **dadurch gekennzeichnet, dass** die Elastizität des mindestens einen Strickfadens und des mindestens einen Schussfadens 1 % bis 400 % beträgt.

7. Strickware (10) nach einem der Ansprüche 1 bis 6, **dadurch gekennzeichnet, dass** der mindestens eine Strickfaden und der mindestens eine Schussfaden eine Feinheit von 44 bis 3.000 Dtex aufweist.

8. Strickware (10) nach einem der Ansprüche 1 bis 7, **dadurch gekennzeichnet, dass** sie unter Anwendung von Spickel- und/oder Umhängetechniken hergestellt ist.

9. Strickware (10) nach einem der Ansprüche 1 bis 8, **dadurch gekennzeichnet, dass** sie Maschen und Fanghenkel unterschiedlicher Größe aufweist.

10. Strickware (10) nach einem der Ansprüche 1 bis 9, **dadurch gekennzeichnet, dass** sie mit angestrickten Applikationen wie Taschen, Ösen, Öffnungen und dergleichen versehen ist.

11. Strickware (10) nach einem der Ansprüche 1 bis 10, **dadurch gekennzeichnet, dass** sie Bereiche mit unterschiedlichen Elastizitäts- oder Kompressionseigenschaften und/oder anderen Festigkeiten und/oder anderen Materialdicken aufweist.

12. Strickware (10) nach Anspruch 11, **dadurch gekennzeichnet, dass** ihre Gestrickabschlüsse (17) andere Elastizitäts- oder Kompressionseigenschaften aufweisen als die übrigen Bereiche.

13. Strickware (10) nach einem der Ansprüche 1 bis 12, **dadurch gekennzeichnet, dass** die oberen Gestrickabschlüsse auf der Innenseite mit eingestrickten Noppen aus Elasthan- oder Elastodienfäden versehen sind.

14. Strickware (10) nach einem der Ansprüche 1 bis 13, **dadurch gekennzeichnet, dass** sie ein Kompressionsarm- oder -beinstrumpf, ein Kompressionshandschuh, eine Kompressionsfußkappe oder eine Kompressionsstrumpfhose ist.

15. Strickware (10) nach einem der Ansprüche 1 bis 13, **dadurch gekennzeichnet, dass** sie eine Verbrennungs- oder Gelenkbandage ist.

16. Strickware (10) nach einem der Ansprüche 1 bis 13, **dadurch gekennzeichnet, dass** sie eine Sportbandage ist.

17. Strickware (10) nach einem der Ansprüche 1 bis 13, **dadurch gekennzeichnet, dass** sie ein Anzug, eine Hose oder eine Jacke ist.

18. Verfahren zur Herstellung einer Strickware (10) zum Stützen und/oder Komprimieren von Körperteilen und/oder zur Kompressionsbehandlung, **dadurch gekennzeichnet, dass** sie mindestens bereichsweise als Schlauchgestrick (11, 12, 13, 14, 15, 16) unter Verwendung eines oder mehrerer elastischer Strickfäden mit einer elastischen Schussfadeneinlage auf einer Flachstrickmaschine gefertigt wird.

19. Verfahren nach Anspruch 18, **dadurch gekennzeichnet, dass** das Schlauchgestrick (11, 12, 13, 14, 15, 16) unter Anwendung von Spickeltechniken und/oder Umhängetechniken und/oder Variation der Maschengröße und/oder Variation der Fadenspannung anatomisch den Körperteilen angepasst wird.

20. Verfahren nach Anspruch 18 oder 19, **dadurch gekennzeichnet, dass** die Form der Körperteile mittels eines Body-Scanning-Verfahrens erfasst und die Flachstrickmaschine mit den Daten über die Körperteilform angesteuert wird.

21. Verfahren nach einem der Ansprüche 18 bis 20, **dadurch gekennzeichnet, dass** der mindestens eine elastische Schussfaden durch Fang in die Strickware (10) eingebunden wird.
